(19) Europäisches Patentamt
European Patent Office
Office européen des brevets

(11) Veröffentlichungsnummer: **0 287 854 A1**

(12) **EUROPÄISCHE PATENTANMELDUNG**

(21) Anmeldenummer: **88105045.4**

(51) Int. Cl.4: **C07D 231/44 , A01N 43/56**

(22) Anmeldetag: **29.03.88**

(30) Priorität: **09.04.87 DE 3712072**

(43) Veröffentlichungstag der Anmeldung:
**26.10.88 Patentblatt 88/43**

(84) Benannte Vertragsstaaten:
**AT BE CH DE ES FR GB GR IT LI NL**

(71) Anmelder: **BAYER AG**
**Konzernverwaltung RP Patentabteilung**
**D-5090 Leverkusen 1 Bayerwerk(DE)**

(72) Erfinder: **Lindig, Markus, Dr.**
**Dahlienweg 16**
**D-4010 Hilden(DE)**
Erfinder: **Becker, Benedikt, Dr.**
**Metzkausener Strasse 14**
**D-4020 Mettmann(DE)**

(54) **1-Aralkylpyrazole.**

(57) Es werden neue 1-Aralkylpyrazole der allgemeinen Formel (I)

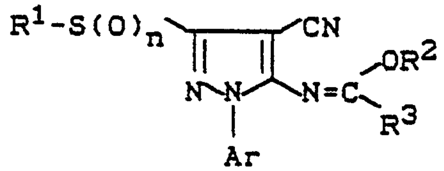

(I)

bereitgestellt, in welcher
$R^1$ für Alkyl steht,
$R^2$ für Alkyl steht,
$R^3$ für Wasserstoff oder Alkyl steht,
Ar für gegebenenfalls substituiertes Aralkyl steht und
n für die Zahlen 0, 1 oder 2 steht.

Die erfindungsgemäßen Verbindungen der Formel (I) werden durch Umsetzung der 5-Aminoverbindungen mit Orthoestern erhalten. Sie besitzen eine sehr gute parasitizide und insbesondere akarizide Wirksamkeit.

EP 0 287 854 A1

## 1-Aralkylpyrazole

Die vorliegende Erfindung betrifft neue 1-Aralkylpyrazole, ein Verfahren zu ihrer Herstellung sowie ihre Verwendung als Schädlingsbekämpfungsmittel.

Es ist bereits bekannt, daß Pyrazolderivate, wie beispielsweise das 1-Cyclohexyl-5-[N,N-(dimethyl)-carbamoyloxy]-3-methylthiomethyl-pyrazol, das 1-Cyclohexyl-5-[N,N-(dimethyl)-carbamoyloxy]-3-methylsulfinylmethyl-pyrazol oder das 1-Cyclohexyl-5-[N,N-(dimethyl)-carbamoyloxy]-3-methylsulfonylmethyl-pyrazol pestizide Eigenschaften besitzen (vgl. z.B. DE-OS 28 39 270).

Die Wirkungshöhe bzw. die Wirkungsdauer dieser Verbindungen sind jedoch insbesondere bei bestimmten Schadorganismen oder bei niedrigen Anwendungskonzentrationen nicht immer völlig zufriedenstellend.

Es wurde neue 1-Aralkylpyrazole der allgemeinen Formel (I),

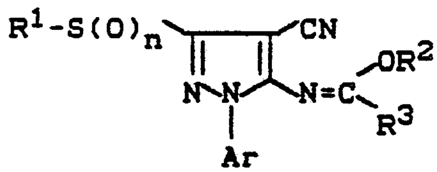

(I)

in welcher
$R^1$ für Alkyl steht,
$R^2$ für Alkyl steht,
$R^3$ für Wasserstoff oder Alkyl steht,
Ar für gegebenenfalls substituiertes Aralkyl steht und
n für die Zahlen 0, 1 oder 2 steht,
gefunden.

Weiterhin wurde gefunden, daß man die neuen 1-Aralkylpyrazole der allgemeinen Formel (I),

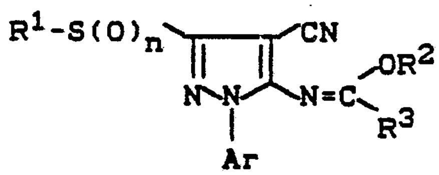

(I)

in welcher
$R^1$ für Alkyl steht,
$R^2$ für Alkyl steht,
$R^3$ für Wasserstoff oder Alkyl steht,
Ar für gegebenenfalls substituiertes Aralkyl steht und
n für die Zahlen 0, 1 oder 2 steht,
erhält, wenn man 5-Amino-1-aralkylpyrazole der Formel (II),

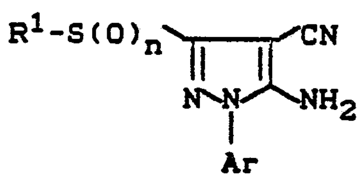

(II)

in welcher
$R^1$, Ar und n die oben angegebene Bedeutung haben,
mit Orthoestern der Formel (III),

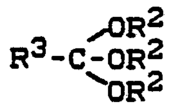

(III)

in welcher
$R^2$ und $R^3$ die oben angegebene Bedeutung haben,
gegebenenfalls in Gegenwart eines Verdünnungsmittels umsetzt.

Schließlich wurde gefunden, daß die neuen 1-Aralkylpyrazole der allgemeinen Formel (I) pestizide, insbesondere akarizide Eigenschaften besitzen.

Überraschenderweise zeigen die erfindungsgemäßen 1-Aralkylpyrazole der allgemeinen Formel (I) eine erheblich bessere akarizide Wirksamkeit als die aus dem Stand der Technik bekannten Pyrazol-Derivate, wie beispielsweise die Verbindungen 1-Cyclohexyl-5-[N,N-(dimethyl)-carbamoyloxy]-3-methylthiomethyl-pyrazol, 1-Cyclohexyl-5-[N,N-(dimethyl)-carbamoyloxy]-3-methylsulfinylmethyl-pyrazol und 1-Cyclohexyl-5-[N,N-(dimethyl)-carbamoyloxy]-3-methylsulfonylmethyl-pyrazol, welche chemisch und wirkungsmäßig naheliegende Verbindungen sind.

Die erfindungsgemäßen 1-Aralkylpyrazole sind durch die Formel (I) allgemein definiert. Bevorzugt sind Verbindungen der Formel (I), bei welchen
$R^1$ für geradkettiges oder verzweigtes Alkyl mit 1 bis 8 Kohlenstoffatomen steht,
$R^2$ für geradkettiges oder verzweigtes Alkyl mit 1 bis 8 Kohlenstoffatomen steht,
$R^3$ für Wasserstoff oder für geradkettiges oder verzweigtes Alkyl mit 1 bis 8 Kohlenstoffatomen steht,
Ar für gegebenenfalls einfach bis mehrfach, gleich oder verschieden substituiertes Phenylalkyl mit 1 bis 4 Kohlenstoffatomen im geradkettigen oder verzweigten Alkylteil steht, wobei als Substituenten des Phenylrestes genannt seien: Halogen, Halogenalkyl oder Halogenalkoxy mit jeweils 1 bis 2 Kohlenstoffatomen und 1 bis 5 gleichen oder verschiedenen Halogenatomen und
n für eine Zahl 0, 1 oder 2 steht.

Besonders bevorzugt sind 1-Aralkylpyrazole der Formel (I), bei welchen
$R^1$ für Methyl, Ethyl, n-oder i-Propyl sowie n-, i-, s-oder t-Butyl steht,
$R^2$ für Methyl, Ethyl, n-oder i-Propyl sowie n-, i-, s-oder t-Butyl steht,
$R^3$ für Wasserstoff, Methyl, Ethyl, n-oder i-Propyl sowie n-, i-, s-oder t-Butyl steht,
Ar für jeweils gegebenenfalls ein-bis dreifach, gleich oder verschieden durch Fluor, Chlor, Trifluormethyl oder Trifluormethoxy substituiertes Benzyl, 1-Phenylethyl oder 2-Phenylethyl steht und
n für eine Zahl 0, 1 oder 2 steht.

Im einzelnen seien außer den bei den Herstellungsbeispielen genannten Verbindungen die folgenden 1-Aralkylpyrazole der allgemeinen Formel (I) genannt:

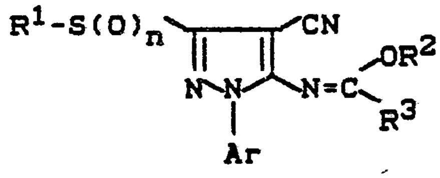

(I)

| $R^1$ | $OR^2$ | $R^3$ | $R^4$ | n |
|---|---|---|---|---|
| $CH_3$ | $-OCH_3$ | H | $-CH_2-$ 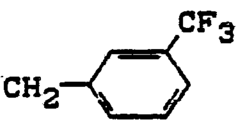 | 0 |
| $CH_3$ | $-OCH_3$ | $CH_3$ | $-CH_2-$ 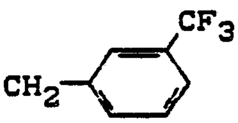 | 0 |
| $CH_3$ | $-OCH_3$ | $CH_3$ | $-CH_2-$ 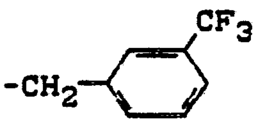 | 2 |
| $CH_3$ | $-OC_2H_5$ | $C_2H_5$ | $-CH_2-$ 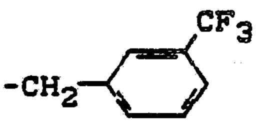 | 2 |

| $R^1$ | $OR^2$ | $R^3$ | $R^4$ | n |
|---|---|---|---|---|
| $CH_3$ | $-OCH_3$ | $C_2H_5$ | 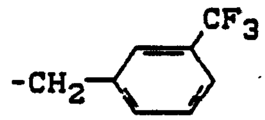 | 0 |
| $CH_3$ | $-OC_2H_5$ | H | 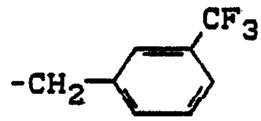 | 1 |
| $CH_3$ | $-OCH(CH_3)_2$ | H | 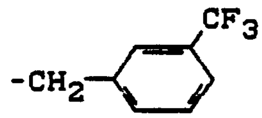 | 0 |
| $CH_3$ | $-OCH(CH_3)_2$ | $CH_3$ | 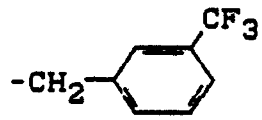 | 0 |
| $CH_3$ | $-OC_2H_5$ | $CH_3$ | 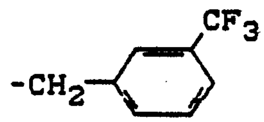 | 1 |
| $C_2H_5$ | $-OC_2H_5$ | $CH_3$ | 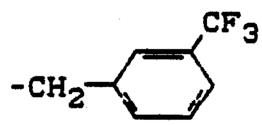 | 0 |
| $CH_3$ | $-OCH_3$ | $CH_3$ | 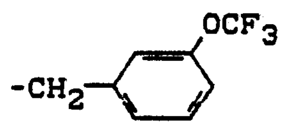 | 0 |
| $CH_3$ | $-OC_2H_5$ | $CH_3$ | 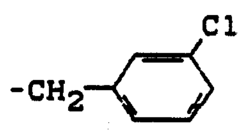 | 0 |
| $CH_3$ | $-OC_2H_5$ | H | 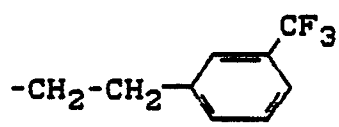 | 0 |
| $CH_3$ | $-OCH_3$ | $CH_3$ | 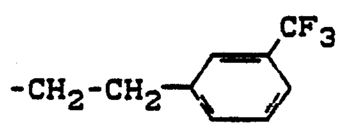 | 0 |
| $CH_3$ | $-OC_2H_5$ | H | 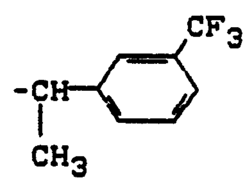 | 0 |

Verwendet man beispielsweise 5-Amino-4-cyano-3-methylthio-1-(3-trifluormethylbenzyl)-pyrazol und Essigsäure-triethylorthoester als Ausgangsstoffe, so läßt sich der Reaktionsablauf des erfindungsgemäßen Verfahrens durch das folgende Formelschema darstellen:

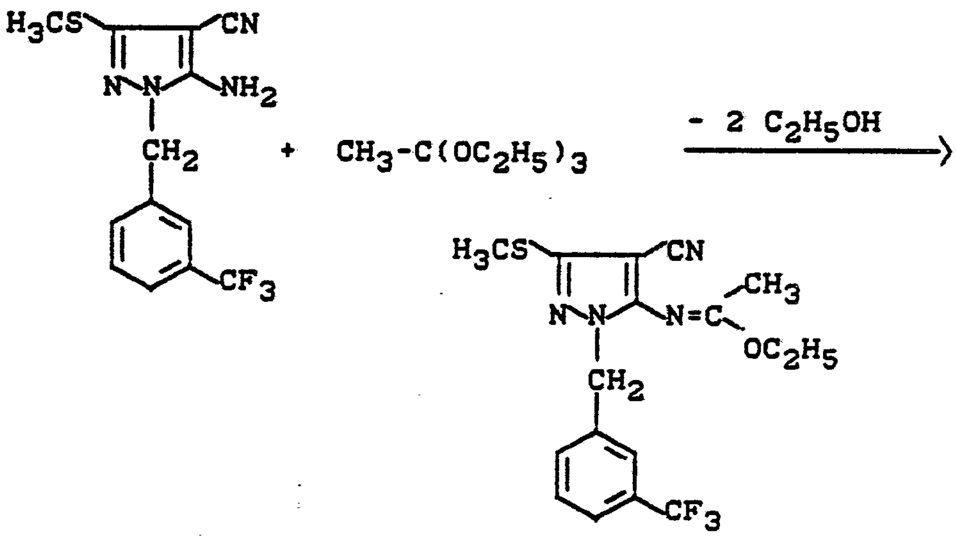


Die zur Durchführung des erfindungsgemäßen Verfahrens als Ausgangsstoffe benötigten 5-Amino-1-aralkylpyrazole sind durch die Formel (II) allgemein definiert. In dieser Formel (II) stehen R[1], Ar und n vorzugsweise für diejenigen Reste und Indices, die bereits im Zusammenhang mit der Beschreibung der erfindungsgemäßen Stoffe der Formel (I) als bevorzugt für diese Substituenten und diesen Index genannt wurden.

Die 5-Amino-1-aralkylpyrazole der Formel (II) sind noch nicht bekannt. Sie sind jedoch Gegenstand einer eigenen noch nicht publizierten Patentanmeldung (vergl. Deutsche Patentanmeldung P 36 16 681). Man erhält sie, wenn man Aralkylhydrazine der Formel (IV),

$Ar - NH - NH_2$ (IV)

in welcher

Ar die oben angegebene Bedeutung hat,

oder deren Säureadditionssalze mit Dinitrilen der Formel (V),

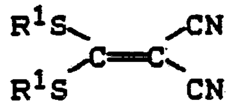
(V)

in welcher

R[1] die oben angegebene Bedeutung hat,

gegebenenfalls in Gegenwart eines Verdünnungsmittels, wie beispielsweise Ethanol bei Temperaturen zwischern 0°C und 60°C umsetzt, und die so erhältlichen 3-Alkylthio-5-amino-1-aralkylpyrazole der Formel (IIa),

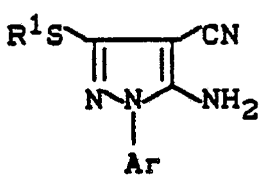
(IIa)

in welcher

R[1] und Ar die oben angegebene Bedeutung haben,

gegebenenfalls mit üblichen Oxidationsmitteln wie beispielsweise m-Chlorperbenzoesäure gegebenenfalls in Gegenwart eines Verdünnungsmittels wie beispielsweise Chloroform bei Temperaturen zwischen 0°C und 40°C am Schwefel in der 3-Position des Pyrazolringes oxidiert.

Die Aralkylhydrazine der Formel (IV) oder deren Säureadditionssalze wie beispielsweise Hydrochloride

sind bekannt (vergl. z.B. DE-OS 22 30 675 oder US-PS 4 370, 339) oder erhältlich in Analogie zu bekannten Verfahren.

Die Dinitrile der Formel (V) sind ebenfalls bekannt (vergl. z.B. Chem.Ber. 95, 2861, 2871 [1962]; Arch. Pharm. 314, 817-823 [1981]; Tetrahedron 30, 3181-3184 [1974] oder DE-OS 32 17 931).

Die zur Durchführung des erfindungsgemäßen Verfahrens weiterhin als Ausgangsstoffe benötigten Orthoester sind durch die Formel (III) allgemein definiert. In dieser Formel (III) stehen $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Orthoester der Formel (III) sind allgemein bekannte Verbindungen der organischen Chemie.

Als Verdünnungsmittel zur Durchführung des erfindungsgemaßen Verfahrens kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Nitrile, wie Acetonitril oder Propionitril oder Amide, wie Dimethylformamid, Dimethylacetamid, N-Methylformanilid, N-Methylpyrrolidon oder Hexamethylphosphorsäuretriamid.

Es ist jedoch auch möglich, den als Reaktionspartner eingesetzen Orthoester der Formel (III) in entsprechendem Überschuß gleichzeitig als Verdünnungsmittel einzusetzen.

Die Reaktionstemperaturen können bei der Durchführung des erfindungsgemäßen Verfahrens in einem größeren Bereich variiert werden. Im allgemeinen arbeitet man bei Temperaturen zwischen 20°C und 150°C, vorzugsweise bei Temperaturen zwischen 50°C und 120°C.

Zur Durchführung des erfindungsgemäßen Verfahrens setzt man pro Mol an 5-Amino-1-aralkylpyrazol der Formel (II) im allgemeinen 1,0 bis 30,0 Mol, vorzugsweise 1,0 bis 15,0 Mol an Orthoester der Formel (III) ein.

Die Reaktionsdurchführung, Aufarbeitung und Isolierung der Reaktionsprodukte erfolgt nach allgemein üblichen Methoden.

Die Wirkstoffe eignen sich zur Bekämpfung von tierischen Schädlingen, vorzugsweise Arthropoden, insbesondere Insekten und Spinnentieren, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella; Plutella maculipennis, Malacosoma neustria, Euproctis chrysorrhoea, Lymantria spp. Bucculatrix thurberiella, Phyllocnistis citrella, Agrotis spp., Euxoa spp., Feltia

spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp, Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.,

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Dabei lassen sich die erfindungsgemäßen Wirkstoffe mit besonders gutem Erfolg zur Bekämpfung der gemeinen Spinnmilbe (Tetranychus urticae) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dipergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharn stoffe, durch Mikrooganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Herstellungsbeispiele:

Beispiel 1:

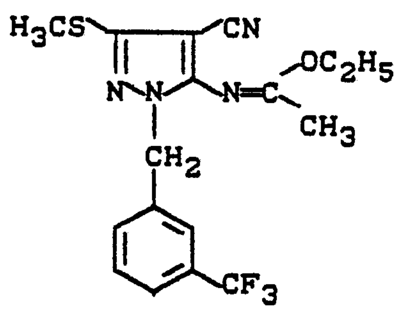

6 g (0,019 Mol) 5-Amino-4-cyano-3-methylthio-1-(3-trifluormethylbenzyl)-pyrazol und 20 ml (17,7 g = 0,11 Mol) Triethylorthoacetat werden bei Rückflußtemperatur 3 Stunden gerührt. Zur Aufarbeitung wird im Vakuum eingeengt und der Rückstand durch Verreiben mit Petrolether zur Kristallisation gebracht. Man erhält 6,0 g (83 % der Theorie) an 4-Cyano-3-methylthio-1-(3-trifluormethylbenzyl-5-(1-ethoxyethylidenimino)-pyrazol vom Schmelzpunkt 65°C.

Herstellung der Ausgangsverbindung

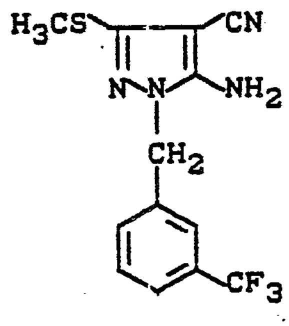

8,5g (0,05 Mol) 1,1-Dicyano-2,2-bis(methylthio)-ethylen und 9,5 g (0,06 Mol) 3-Trifluormethyl-benzyl-hydrazin (vergl. DE-OS 22 30 675) werden in 100 ml Ethanol 15 Stunden lang bei 20° C gerührt. Zur Aufarbeitung entfernt man das Lösungsmittel im Vakuum und verreibt den Rückstand mit Petrolether. Nach Absaugen erhält man 12,4 g (80% der Theorie) an 5-Amino-4-cyano-3-methylthio-1-(3-trifluormethylbenzyl)-pyrazol vom Schmelzpunkt 142°C.

In entsprechender Weise und gemäß den allgemeinen Angaben zur Herstellung erhält man die folgenden 1-Aralkylpyrazole der allgemeinen Formel (I):

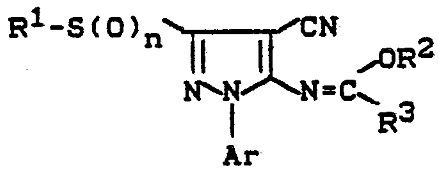

(I)

| Bsp. Nr. | $R^1$ | $OR^2$ | $R^3$ | Ar | n | Schmelz-punkt/° C |
|---|---|---|---|---|---|---|
| 2 | $CH_3$ | $-OC_2H_5$ | H | 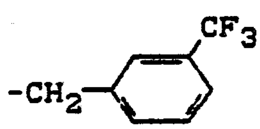 | 0 | 82 |
| 3 | $CH_3$ | $-OC_2H_5$ | $C_2H_5$ | 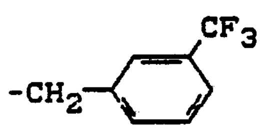 | 0 | 54 |

Anwendungsbeispiele:

In dem folgenden Anwendungsbeispiel wurden die nachstehend aufgeführten Verbindungen als Vergleichssubstanzen eingesetzt:

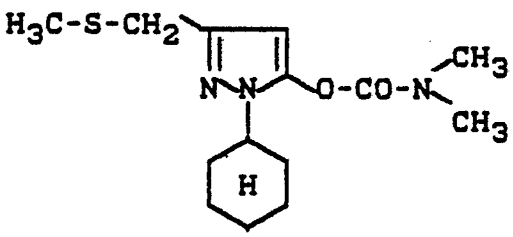

(A)

1-Cyclohexyl-5-(N,N-dimethylcarbamoyloxy)-4-methyl-thiomethyl-pyrazol

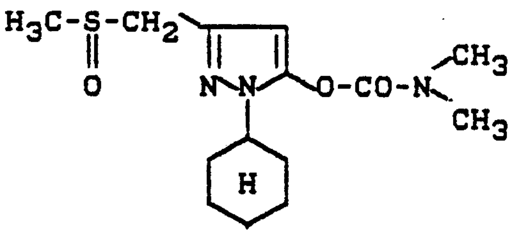

(B)

1-Cyclohexyl-5-(N,N-dimethylcarbamoyloxy)-3-methyl sulfinylmethyl-pyrazol

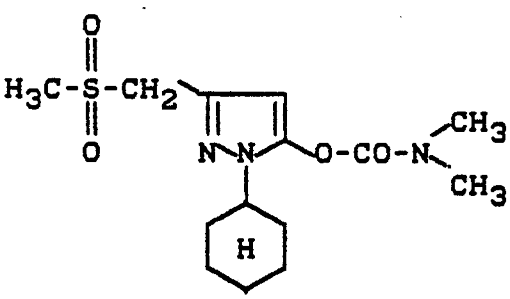

(C)

1-Cyclohexyl-5-(N,N-dimethylcarbamoyloxy)-3-methyl sulfonylmethyl-pyrazol

(alle bekannt aus DE-OS 28 39 270)

Beispiel A

Tetranychus-Test (resistent)

Lösungsmittel: 7 Gewichtsteile Dimethylformamid
Emulgator: 1 Gewichtsteil Alkylarylpolyglykolether
Zur Herstellung einer zweckmäßigen Wirkstoffzubereitung vermischt man 1 Gewichtsteil Wirkstoff mit

der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet werden; 0 % bedeutet, daß keine Spinnmilben abgetötet werden.

Bei diesem Test zeigen z.B. die folgenden Verbindungen der Herstellungsbeispiele überlegene Wirksamkeit gegenüber dem Stand der Technik: 1, 2 und 3.

**Ansprüche**

1. 1-Aralkylpyrazole der allgemeinen Formel (I)

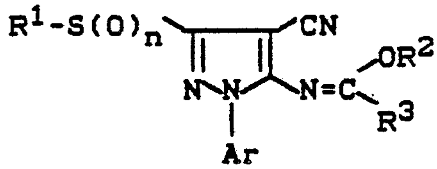

(I)

in welcher
$R^1$ für Alkyl steht,
$R^2$ für Alkyl steht,
$R^3$ für Wasserstoff oder Alkyl steht,
Ar für gegebenenfalls substituiertes Aralkyl steht und
n für die Zahlen 0, 1 oder 2 steht.

2. 1-Aralkylpyrazole der allgemeinen Formel (I) gemäß Anspruch 1, in welcher
$R^1$ für geradkettiges oder verzweigtes Alkyl mit 1 bis 8 Kohlenstoffatomen steht,
$R^2$ für geradkettiges oder verzweigtes Alkyl mit 1 bis 8 Kohlenstoffatomen steht,
$R^3$ für Wasserstoff oder für geradkettiges oder verzweigtes Alkyl mit 1 bis 8 Kohlenstoffatomen steht,
Ar für gegebenenfalls einfach bis mehrfach, gleich oder verschieden substituiertes Phenylalkyl mit 1 bis 4 Kohlenstoffatomen im geradkettigen oder verzweigten Alkylteil steht,
n für eine Zahl 0, 1 oder 2 steht.

3. 1-Aralkylpyrazole der allgemeinen Formel (I) gemäß Anspruch 1, in welcher
$R^1$ für Methyl, Ethyl, n-oder i-Propyl sowie n-, i-, s-oder t-Butyl steht,
$R^2$ für Methyl, Ethyl, n-oder i-Propyl sowie n-, i-, s-oder t-Butyl steht,
$R^3$ für Wasserstoff, Methyl, Ethyl, n-oder i-Propyl sowie n-, i-, s-oder t-Butyl steht,
Ar für jeweils gegebenenfalls ein-bis dreifach, gleich oder verschieden durch Fluor, Chlor, Trifluormethyl oder Trifluormethoxy substituiertes Benzyl, 1-Phenylethyl oder 2-Phenylethyl steht und
n für eine Zahl 0, 1 oder 2 steht.

4. Verfahren zur Herstellung von 1-Aralkylpyrazolen der allgemeinen Formel (I),

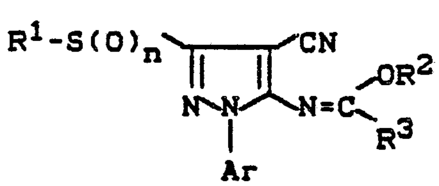

(I)

in welcher
$R^1$ für Alkyl steht,
$R^2$ für Alkyl steht,
$R^3$ für Wasserstoff oder Alkyl steht,

Ar für gegebenenfalls substituiertes Aralkyl steht und
n für die Zahlen 0, 1 oder 2 steht,
dadurch gekennzeichnet, daß man 5-Amino-1-aralkylpyrazole der Formel (II)

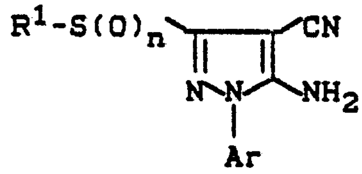

(II)

in welcher
$R^1$, Ar und n die oben angegebene Bedeutung haben,
mit Orthoestern der Formel (III),

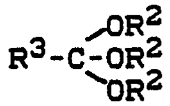

(III)

in welcher
$R^2$ und $R^3$ die oben angegebene Bedeutung haben,
gegebenenfalls in Gegenwart eines Verdünnungsmittels umsetzt.

5. Schädlingsbekämpfungsmittel, gekennzeichnet durch einen Gehalt an mindestens einem 1-Aralkylpyrazol der Formel (I).

6. Verfahren zur Bekämpfung von tierischen Schädlingen, dadurch gekennzeichnet, daß man 1-Aralkylpyrazole der Formel (I) auf tierische Schädlinge und/oder ihren Lebensraum einwirken läßt.

7. Verwendung von 1-Aralkylpyrazolen der Formel (I) zur Bekämpfung von tierischen Schädlingen.

8. Verfahren zur Herstellung von Schädlingsbekämpfungsmitteln, dadurch gekennzeichnet, daß man 1-Aralkylpyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88105045.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | DE - A1 - 3 616 681 (BAYER AG)<br>* Ansprüche 1,4,5-9 *<br>---- | 1,4,5-8 | C 07 D 231/44<br>A 01 N 43/56 |
| | | | **RECHERCHIERTE SACHGEBIETE (Int. Cl.4)** |
| | | | C 07 D 231/00 |

Der vorliegende Recherchenbericht wurde für alle Patentansprüche erstellt.

| Recherchenort | Abschlußdatum der Recherche | Prüfer |
|---|---|---|
| WIEN | 29-06-1988 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82